# EUROPÄISCHE PATENTSCHRIFT

(11) **EP 3 318 105 B1**
(45) Veröffentlichungstag und Bekanntmachung des Hinweises auf die Patenterteilung: **04.12.2019**
(21) Anmeldenummer: 16781053.0
(22) Anmeldetag: 26.09.2016
(51) Int. Cl.: H05H 1/24, A61L 2/14

(54) **ELEKTRODENANORDNUNG FÜR EINE DIELEKTRISCH BEHINDERTE PLASMABEHANDLUNG**
ELECTRODE ARRAY FOR A DIELECTRIC BARRIER DISCHARGE PLASMA TREATMENT
AGENCEMENT D'ÉLECTRODES POUR UN TRAITEMENT AU PLASMA PAR DÉCHARGE À BARRIÈRE DIÉLECTRIQUE

(30) Priorität: 19.10.2015 DE 102015117715
(43) Veröffentlichungstag der Anmeldung: 09.05.2018
(73) Patentinhaber: Cinogy GmbH, 37115 Duderstadt (DE)
(72) Erfinder: TRUTWIG, Leonhard, 37115 Duderstadt/Gerlingerode (DE); HAHNL, Mirko, 37339 Berlingerode (DE); STORCK, Karl-Otto, 37115 Duderstadt (DE); WANDKE, Dirk, 37308 Heilbad Heiligenstadt (DE); KOPP, Matthias, 37434 Gieboldehausen (DE)
(74) Vertreter: Lins, Martina
(86) Internationale Anmeldenummer: PCT/DE2016/100446
(87) Internationale Veröffentlichungsnummer: WO 2017/067535

(56) Entgegenhaltungen:
- EP-A1- 2 883 426
- WO-A1-2013/040542
- WO-A1-2015/070832
- KR-B1- 101 407 672
- US-A1- 2006 042 545
- US-A1- 2013 345 620

## Beschreibung

Die Erfindung betrifft eine Elektrodenanordnung für eine dielektrisch behinderte Plasmabehandlung einer als Gegenelektrode verwendeten Oberfläche eines elektrisch leitenden Körpers, mit einer flexiblen flächigen Elektrode und einem Dielektrikum aus einem flächigen flexiblen Material, das mit einer einen direkten Stromfluss verhindernden Schicht die Elektrode von der zu behandelnden Oberfläche abschirmt, wobei das Dielektrikum über eine Struktur mit Vorsprüngen, auf der zu behandelnden Oberfläche aufliegen kann und dabei zwischen den Vorsprüngen Lufträume für die Ausbildung des Plasmas ausgebildet sind, wobei die Struktur eine Gitterstruktur ist, die Lufträume ausbildende Kammern aufweist und die Kammern einen bodenseitigen Abschluss durch die den direkten Stromfluss verhindernde Schicht des Dielektrikums und eine zur zu behandelnden Oberfläche offene Seite aufweisen.

Eine Elektrodenanordnung für eine dielektrisch behinderte Plasmabehandlung ist durch DE 10 2009 060 627 B4 bekannt. Deren Aufbau ermöglicht die Ausbildung einer flächigen flexiblen Elektrodenanordnung, die an auch unregelmäßig gewölbte Oberflächen anpassbar ist, sodass die Elektrodenanordnung auf diese Oberfläche zur Durchführung einer Plasmabehandlung aufgelegt werden kann. Damit dabei ein Plasma entstehen kann, ist das Dielektrikum mit der Vorsprünge aufweisenden Struktur ausgebildet, mit denen die Elektrodenanordnung auf der Oberfläche aufliegen und sich dennoch ein Plasma in den Lufträumen zwischen den Vorsprüngen ausbilden kann. Unter "Lufträumen" werden dabei und im Rahmen dieser Anmeldung Leerräume verstanden, die üblicherweise mit Luft gefüllt sind, aber auch für bestimmte Anwendungsfälle mit einem geeigneten Gas gefüllt werden können, um spezielle Plasmen auszubilden. Die mit den Vorsprüngen gebildete Struktur des Dielektrikums kann mit der den direkten Stromfluss von der Elektrode verhindernden Schicht einstückig ausgebildet oder als separates Bauelement hergestellt sein, das mit der Schicht mechanisch, formschlüssig und/oder stoffschlüssig verbunden werden kann. Die flächige flexible Elektrode ist in das Dielektrikum vorzugsweise vollständig eingebettet, wobei das Dielektrikum aus zwei Lagen bestehen kann, zwischen denen die Elektrode - mit kleinerer flächiger Ausdehnung - eingelegt wird, woraufhin die beiden Lagen des Dielektrikums miteinander verbunden werden. Dies kann stoffschlüssig durch Aufschmelzen des Materials des Dielektrikums im Bereich der Trennflächen, aber auch durch Verwendung eines geeigneten isolierenden Klebers erfolgen. In einer anderen Ausführungsform wird die flächige Elektrode, die aus einem Drahtgitter gebildet sein kann, mit dem Material des Dielektrikums zur Formgebung für die Elektrodenanordnung beim Spritzgießen oder in einem Gießverfahren umhüllt.

Die bekannte Elektrodenanordnung hat sich bewährt und ist insbesondere auch für die Behandlung der Hautoberfläche eines menschlichen oder tierischen Körpers geeignet. Durch die Plasmabehandlung können therapeutische oder kosmetische Wirkstoffe verbessert aufgenommen werden, sodass die Plasmabehandlung die angestrebte therapeutische oder kosmetische Wirkung verstärkt. Die Plasmabehandlung sorgt ferner für eine wirksame Desinfektion, da sie Mikroorganismen zerstört und insbesondere eine bakterizide und fungizide Wirkung auf der Haut ausübt.

Für die Behandlung der Haut mit medizinischen oder kosmetischen Wirkstoffen ist es naheliegend, dass diese in Kombination mit der Elektrodenanordnung auf die Haut aufgebracht werden.

In US 2013/0345620 A1 ist in Figur 10 ein Aufbau eines flexiblen Plasmaemitters mit mehreren Lagen offenbart, in denen ein flächiger zentraler Leiter beidseitig mit einer Dielektrikumslage abgedeckt ist. Eine weitere flächige Lage enthält fensterartige Ausschnitte, die Ausnehmungen darstellen, in denen sich ein Plasma ausbilden kann, wenn die Anordnung mit der mit den Ausschnitten versehenen Lage an der Haut eines Patienten anliegt. Die fensterartigen Ausschnitte stellen einen nur relativ kleinen Raum für eine Plasmaausbildung zur Verfügung.

Der vorliegenden Erfindung liegt die Problemstellung zugrunde, die bewährte bekannte Elektrodenanordnung mit den durch sie erzielbaren Vorteilen beizubehalten und hinsichtlich ihrer Fertigbarkeit und ihrer Verwendbarkeit im kosmetischen oder medizinischen Bereich noch zu verbessern.

Zur Lösung dieser Aufgabe ist eine Elektrodenanordnung der eingangs erwähnten Art erfindungsgemäß dadurch gekennzeichnet, dass die Kammern durch aneinander angrenzende Wände mit einer Materialstärke zwischen 0,1 und 1,0 mm und einer Höhe von 0,5 bis 3 mm über der den direkten Stromfluss verhindernden Schicht des Dielektrikums begrenzt sind.

Die Ausbildung der die Lufträume definierenden Struktur der Elektrodenanordnung als Gitterstruktur ermöglicht eine Einhaltung des Abstandes zwischen der durchgehenden, den direkten oder galvanischen Stromfluss von der Elektrode verhindernden Schicht des Dielektrikums zur zu behandelnden Oberfläche mit einer sehr flexiblen und leichten Struktur, die vorzugsweise aus einem nicht resorbierenden Material besteht, also Flüssigkeiten nicht aufnimmt. Geeignete Materialien sind dabei flexible Silikone, insbesondere Silikone, die unter dem Handelsnamen SILPURAN® von der Firma Wacker Chemie vertrieben werden. Hierdurch eignet sich die erfindungsgemäße Elektrodenanordnung auch für die Behandlung von Oberflächen, bei der Flüssigkeiten an der Oberfläche vorhanden sind oder entstehen, wie dies bspw. bei einer Wunde auf der Haut der Fall sein kann. Die erfindungsgemäße Elektrodenanordnung eignet sich somit auch als Wundauflage, da das Material sich nicht mit der Wunde oder mit Wundsekret verbindet, wodurch beim Entfernen der Elektrodenanordnung ein Aufreißen einer entstandenen Heilschicht resultieren würde.

In einer bevorzugten Ausführungsform der Erfindung bilden - vorzugsweise zwei - Wandscharen mit winkelig zueinander stehenden Wänden Kammern als Lufträume aus, die durch zueinander kreuzende Wandpaare begrenzt sind. Dabei wird die erfindungsgemäße Gitterstruktur vorzugsweise durch zwei Scharen von jeweils parallel zueinander verlaufenden Wänden gebildet, wobei sich die Wände der beiden Scharen kreuzen. Hierdurch können rechteckige, aber auch rautenförmige Kammern als Lufträume entstehen, die durch jeweils zwei Wände der beiden Scharen begrenzt werden. Das Material der Gitterstruktur ist grundsätzlich beliebig, kann beispielsweise auch mehr oder weniger elektrisch leitend sein. Bevorzugt ist jedoch eine Gitterstruktur aus einem dielektrischen Material, das auch mit dem Material des Dielektrikums identisch oder ähnlich sein kann.

Die Ausbildung der Kammern durch zwei Scharen von jeweils parallel zueinander verlaufenden Wänden hat den Vorteil, dass immer gleich große Kammern gebildet werden, die von Wänden mit einer einheitlichen Wandstärke begrenzt sind. Der gleiche Effekt lässt sich auch erreichen, wenn die Gitterstruktur eine Wabenstruktur aus sechseckigen Waben ist.

In einer bevorzugten Ausführungsform stehen die beiden Scharen von Wänden senkrecht zueinander, sodass rechteckige, bevorzugt quadratische, Kammern gebildet werden.

Erfindungsgemäß kann die Gitterstruktur aber auch Kammern mit einem runden, ovalen oder vieleckigen Querschnitt aufweisen. Die aneinander grenzenden Wände dieser Kammern bilden dann Zwickel aus, die ebenfalls als Lufträume dienen können, sodass die Gitterstruktur Kammern mehrerer Größen aufweist, insbesondere Kammern zweier Größen. Dabei ist es allerdings auch möglich, die Zwickelräume mit dem Wandmaterial auszufüllen, um die Stabilität der Gitterstruktur zu erhöhen. In diesem Fall werden die Kammern durch Wände mit einer gleichen Grundwandstärke begrenzt, die jedoch im Zwickelbereich verdickt ausgebildet sind.

Die Wände haben vorzugsweise eine identische Höhe, sodass Kammern gebildet werden, die seitlich geschlossen sind und einen abgeschlossenen Luftraum bilden, wenn die Elektrodenanordnung auf die zu behandelnde Oberfläche aufgelegt ist. Untersuchungen haben ergeben, dass auch in derartigen abgeschlossenen Kammern ein geeignetes Plasma ausgebildet werden kann. Dies gilt sogar dann, wenn die Kammern mit einem Behandlungsmaterial teilweise gefüllt werden. Das Material kann in Salben und Pastenform, aber auch als resorbierbarer poröser Feststoff in die Kammern eingebracht werden, wobei weiterhin ein geeignetes Plasma erzeugbar ist, wenn die Kammern nicht vollständig gefüllt sind.

Die erfindungsgemäße Gitterstruktur ist besonders vorteilhaft, wenn die Materialstärke der Wände weniger als 20 %, vorzugsweise weniger als 10 %, der größten Breite einer Kammer ausmacht. Auf diese Weise steht ein sehr großes Volumen für die Plasmaausbildung zur Verfügung, wobei die Gitterstruktur dennoch für eine sichere Abstandshaltung sorgt.

Die Materialstärke der Wände liegt zwischen 0,1 und 1,0 mm, insbesondere zwischen 0,4 und 0,6 mm. Eine gewünschte Stabilität der Abstandshaltung durch die Gitterstruktur wird auch bei sehr flexiblem Material erreicht, wenn dabei die Höhe der Wände über der den direkten Stromfluss verhindernden Schicht des Dielektrikums zwischen 0,5 und 3 mm, insbesondere zwischen 1 und 2 mm liegt.

Die Gitterstruktur kann einstückig mit der den Stromkreis verhindernden Schicht des Dielektrikums ausgebildet sein. Dieser Ausbildung ist im Gießverfahren herstellbar. Die erfindungsgemäße Gitterstruktur ermöglicht aber auch den schnellen Aufbau nach Art eines Prototyps im 3D-Druckverfahren.

Die erfindungsgemäße Gitterstruktur kann in der gleichen Weise, also im Gießverfahren oder im 3D-Druckverfahren, auch als separates Teil gefertigt werden, um dann an die den direkten oder galvanischen Stromfluss verhindernde Schicht angesetzt zu werden. Die Herstellung einer festen Verbindung zwischen der Gitterstruktur und der Schicht des Dielektrikums kann dann in üblicher Weise erfolgen, also mechanisch in einer Gehäusestruktur, formschlüssig und/oder stoffschlüssig, letzteres durch Kleben oder Schweißen. Die separate Herstellung der Gitterstruktur kann insbesondere bei der Behandlung von Wunden den Vorteil haben, eine leichte Austauschbarkeit des mit der Wunde in Berührung gelangenden Teils der Elektrodenanordnung zu ermöglichen, wobei das separate Teil als entfernbares Einmalteil verwendbar ist oder auch wegen des geringen Volumens einfach sterilisiert werden kann.

Insbesondere als für eine Wundbehandlung geeignete Elektrodenanordnung ist eine Ausführungsform bevorzugt, bei der die flächige Elektrode über ihre Fläche verteilte Durchgangsöffnungen aufweist und sich das die Elektrode abschirmende Dielektrikum auf beiden Seiten der flächigen Elektrode erstreckt und mit zur Ableitung von Fluid von der zu behandelnden Oberfläche ausgebildeten Durchgangsöffnungen versehen ist, die mit den Durchgangsöffnungen der Elektrode fluchten und kleinere Abmessungen als die Durchgangsöffnungen der Elektrode aufweisen, sodass das Dielektrikum auch im Bereich der Durchgangsöffnungen die Elektrode vollständig abdeckt. Durch diese Durchgangsöffnungen kann Wundsekret abgeführt werden, ohne dass die Gefahr besteht, dass über das elektrische leitende Wundsekret ein Durchschlag von der Elektrode auf die Haut erfolgt. Die Durchgangsöffnungen können ferner dazu verwendet werden, auf die zu behandelnde Oberfläche einen Gasstrom, gegebenenfalls auch als Luftstrom, zu leiten. Jedenfalls ist es zweckmäßig, die Durchgangsöffnungen im Bereich der durchgehenden Schicht mit Kammern der Gitterstruktur fluchten zu lassen.

Die erfindungsgemäße Gitterstruktur kann zum direkten Kontakt mit einer Wunde geeignet sein. Es ist jedoch auch möglich, auf die Gitterstruktur eine dünne Schicht aus einem Wundauflagenmaterial aufzubringen, das eine vollwertige Wundauflage darstellt. Das Wundauflagenmaterial kann sich auch innerhalb der Gitterstruktur befinden, wenn es offenporig ist und somit die Ausbildung des Plasmas innerhalb der Gitterstruktur ermöglicht.

Die Erfindung soll im Folgenden anhand von in der Zeichnung dargestellten Ausführungsbeispielen näher erläutert werden. Es zeigen:
- Figur 1: einen Vertikalschnitt durch ein erstes Ausführungsbeispiel einer erfindungsgemäßen Elektrodenanordnung;
- Figur 2: einen Vertikalschnitt durch eine Modifikation der Elektrodenanordnung gemäß der ersten Ausführungsform;
- Figur 3: einen Horizontalschnitt in Höhe der flachen Elektrode der Elektrodenanordnung der ersten Ausführungsform;
- Figur 4: einen Vertikalschnitt durch eine Elektrodenanordnung gemäß einem zweiten Ausführungsbeispiel;
- Figur 5: eine schematische explodierte Darstellung der Elektrodenanordnung gemäß der zweiten Ausführungsform;
- Figur 6: eine schematische Draufsicht auf die Struktur einer Elektrodenanordnung nach einer dritten Ausführungsform;
- Figur 7: eine schematische Draufsicht auf die Struktur einer Elektrodenanordnung nach einer vierten Ausführungsform;
- Figur 8: eine schematische Draufsicht auf die Struktur einer Elektrodenanordnung nach einer fünften Ausführungsform;
- Figur 9: eine schematische Draufsicht auf die Struktur einer Elektrodenanordnung nach einer sechste Ausführungsform;
- Figur 10: eine schematische Draufsicht auf die Struktur einer Elektrodenanordnung nach einer siebten Ausführungsform.

Der Vertikalschnitt durch eine Elektrodenanordnung gemäß einer ersten Ausführungsform zeigt eine metallische, flächige und flexible Elektrode 1, die allseitig von einem Dielektrikum 2 umgeben ist. Insbesondere bildet das Dielektrikum eine zu einer zu behandelnden Oberfläche zeigende untere Schicht 3 und eine von der zu behandelnden Oberfläche weg zeigende obere Schicht 4 aus. Das Dielektrikum 2 ist allseitig größer dimensioniert als die Elektrode 1, wodurch sich ergibt, dass das Dielektrikum 2 die Elektrode 1 allseitig abdeckt. Insbesondere stellt die untere Schicht 3 eine den direkten (galvanischen) Stromfluss zwischen Elektrode 1 und der (nicht dargestellten) zu behandelnden Oberfläche verhindernde Schicht 3 dar.

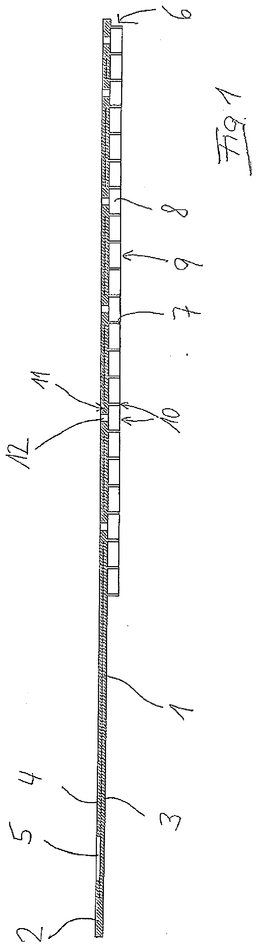

Figur 1 lässt erkennen, dass die obere Schicht 4 an einem Randbereich der Elektrode 1 eine Aussparung 5 aufweist, über die eine Hochspannung der Elektrode 1 zuführbar ist.

An die untere Schicht 3 schließt sich an das Dielektrikum einstückig eine Struktur 6 in Form einer Gitterstruktur an, die durch parallel zueinander verlaufende Wände 7, 8 gebildet ist, wobei die Wände 7 einerseits und Wände 8 andererseits jeweils parallel zueinander verlaufen und die Wände 7 und 8 senkrecht zueinander stehen. Zwischen je zwei Paaren der Wände 7 und 8 entsteht jeweils eine rechteckige, vorzugsweise quadratische, Kammer 9, die bodenseitig durch die untere, den direkten Stromfluss von der Elektrode 1 verhindernde Schicht 3 abgeschlossen ist. Zur anderen Seite hin sind die Kammern 9 offen. Wenn die Wände 7, 8, die vorzugsweise gleich hoch ausgebildet sind, an der zu behandelnden Oberfläche anliegen, bilden ihre Endkanten 10 somit die gitterartige Anlagefläche an der zu behandelnden Oberfläche.

Figur 1 lässt ferner erkennen, dass die Elektrode 1 Durchgangsöffnungen 11 aufweist, in denen sich zentriert eine kleinere Durchgangsöffnung 12 des Dielektrikums 2 befindet. Die Durchgangsöffnung 11 der Elektrode 1 wird somit nahezu vollständig, nämlich bis auf die kleinere Durchgangsöffnung 12 ausgefüllt, sodass auch im Bereich der Durchgangsöffnung 12 die Elektrode 1 vollständig durch das Dielektrikum 2 abgeschirmt ist.

Die Durchgangsöffnungen 11, 12 fluchten vorzugsweise mit jeweils einer Kammer 9 und ermöglichen die Abführung eines Fluids, insbesondere einer Flüssigkeit, von der zu behandelnden Oberfläche durch die betreffende Kammer 9 hindurch.

Die so ausgebildete Elektrodenanordnung ist insbesondere für die Behandlung einer mit einer Wunde versehenen Hautoberfläche geeignet, wobei ein Wundsekret durch die Durchgangsöffnungen 12 auf die distale Oberfläche der Elektrodenanordnung abführbar ist.

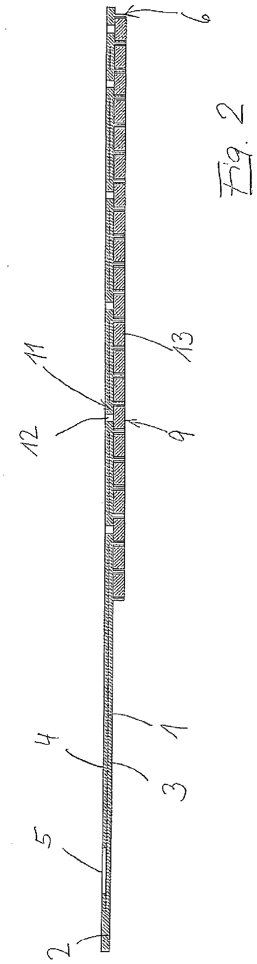

Die in Figur 2 dargestellte Modifikation der in Figur 1 dargestellten Ausführungsform der Elektrodenanordnung zeigt lediglich, dass die Kammern 9 mit einem Material 13 gefüllt sein können, nämlich mit einer heilenden oder hautpflegenden Substanz, beispielsweise Collagen, oder mit einem flüssigkeitsaufsaugenden Material, das watteähnlich ausgebildet sein kann und daher in den Kammern 9 noch einen ausreichenden Luftraum für die Ausbildung des Plasmas gewährleistet. Ein nicht poröses oder nicht faseriges Material, wie Collagen, sollte die Kammern 9 nur teilweise füllen, damit ein ausreichender Luftraum für die Ausbildung des Plasmas bestehen bleibt.

Die Bildung des Dielektrikums 2 mit der Gitterstruktur 6 aus einem hydrophoben Material, wie beispielweise einem hautfreundlichen geeigneten Silikon, ermöglicht die direkte Auflage der Elektrodenanordnung auf die Hautoberfläche bzw. eine Wunde. In dem Fall liegen die Endkanten 10 der Wände 7, 8 direkt auf der Hautoberfläche bzw. der Wunde auf. Durch das hydrophobe Material wird ein Verkleben der Gitterstruktur 6 mit Wundsekret vermieden, sodass die Elektrodenanordnung von der Wunde abnehmbar ist, ohne die Wunde wieder aufzureißen.

Es ist aber auch möglich, auf die Endkanten 10 der Wände 7, 8 der Gitterstruktur 6 ein Wundauflagematerial, beispielsweise in Form einer Gazeschicht, aufzubringen, um dadurch eine sterile Wundauflage zu gewährleisten.

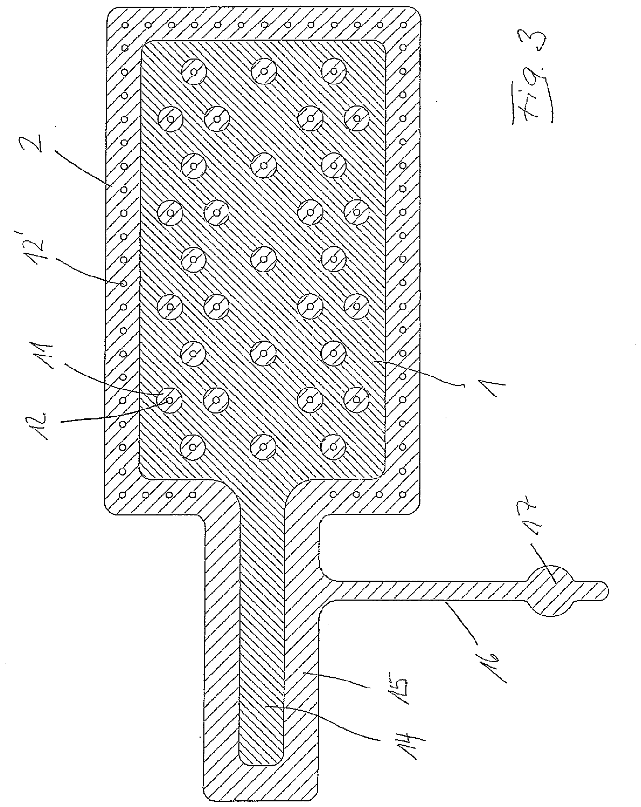

Die Darstellung in Figur 3 eines Horizontalschnitts durch die Elektrodenanordnung gemäß Figur 1 lässt eine Formgebung erkennen, in der die Elektrode 1 einen Anschlusslasche 14 ausbildet, an der die Kontaktierung durch die Aussparung 5 hindurch erfolgen kann. Auch im Bereich der Anschlusslasche 14 ist die Elektrode 1 allseitig - mit Ausnahme der Aussparung 5 - von dem Dielektrikum 2 umgeben, sodass auch das Dielektrikum eine Laschenstruktur 15 bildet. An die Laschenstruktur 15 ist ein flexibler Streifen 16 mit einem zylindrischen Verschlussteil 17 angeformt. Die Größe des zylindrischen Verschlussteils 17 entspricht der Größe der Ausnehmung 5 und dient dem Verschluss der Ausnehmung 5, wenn die Elektrodenanordnung nicht kontaktiert wird.

Figur 3 lässt erkennen, dass das Dielektrikum die im Wesentlichen rechteckig ausgebildete Elektrode 1 rahmenförmig umgibt und allseitig über die Elektrode 1 übersteht. In dem dargestellten Ausführungsbeispiel sind in dem streifenförmigen, über die Elektrode 1 überstehenden Bereich des Dielektrikums 2 weitere Durchgangsöffnungen 12' angeordnet, die ebenfalls der Abführung von gasförmigen oder flüssigen Stoffen von der zu behandelnden Oberfläche, insbesondere von Wundsekret aus einer Wunde, dienen.

Es ist erkennbar, dass der Bereich der Elektrodenanordnung, in der sich die Anschlusslasche 14 der Elektrode 1 und die Laschenstruktur 15 des Dielektrikums 2 befinden, nicht zur Auflage auf der zu behandelnden Oberfläche vorgesehen sind, sodass sich in diesem Bereich gemäß den Figuren 1 und 2 auch keine Gitterstruktur 6 befindet.

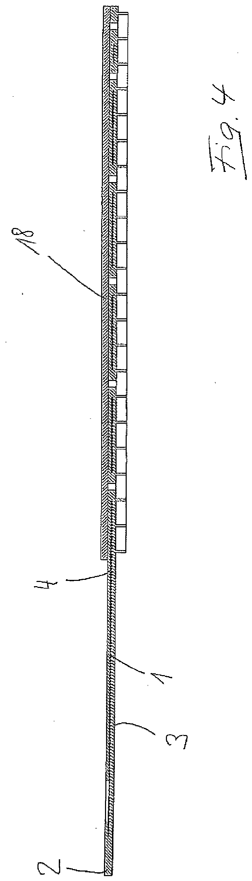

Figur 4 zeigt einen der Figur 1 entsprechenden Vertikalschnitt durch eine zweite Ausführungsform einer erfindungsgemäßen Elektrodenanordnung. Diese Ausführungsform ist identisch mit der in Figur 1 dargestellten Ausführungsform und weist lediglich eine auf die obere Schicht 4 des Dielektrikums oberhalb der Gitterstruktur 6 aufgebrachte Lage 18 aus einem wundsekretabsorbierenden Material auf. Diese Lage 18 kann mit dem Dielektrikum 2 durch Klebung o. ä. verbunden werden, aber auch Teil eines Sekundärverbands sein, mit dem die Elektrodenanordnung auf der zu behandelnden Oberfläche fixiert wird.

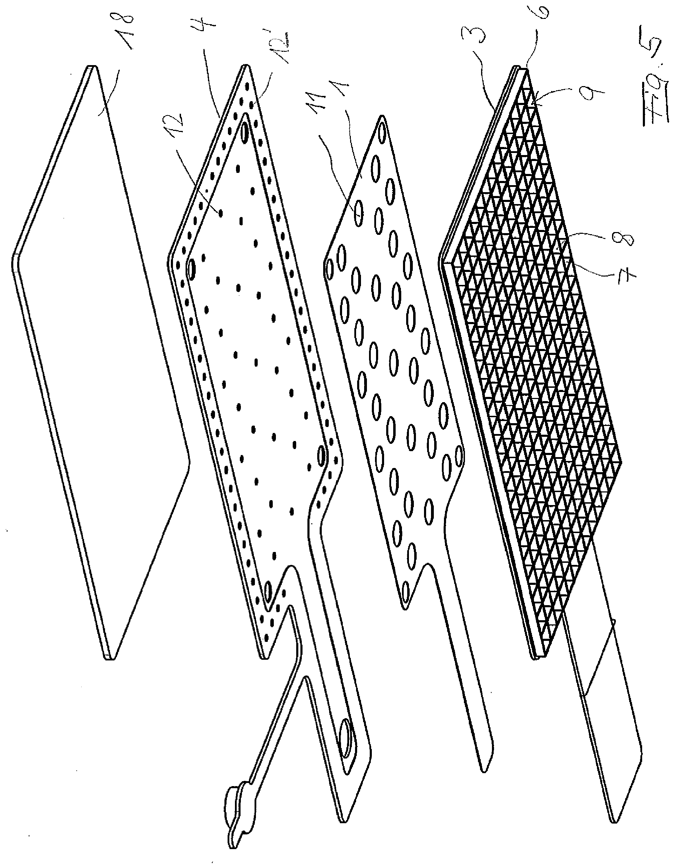

Der Aufbau der Elektrodenanordnung gemäß der zweiten Ausführungsform wird anhand der explodierten Darstellung gemäß Figur 5 verdeutlicht. Auf der der zu behandelnden Oberfläche abgewandten Seite befindet sich die Lage 18 aus dem wundsekretabsorbierenden Material. Wird diese Lage 18 fortgelassen, ergibt sich die in Figur 1 dargestellte erste Ausführungsform der Elektrodenanordnung.

Unterhalb der Lage 18 befindet sich die obere Schicht 4 des Dielektrikums 2, das mit einer Innenkontur 19 die flächige Elektrode 1 aufnimmt. Auf der anderen Seite der Elektrode 1 ist die untere Schicht 3 des Dielektrikums 2 mit der darauf angebrachten Gitterstruktur 6 aus den sich kreuzenden Wänden 7, 8 ausgebildet.

Die explodierte Darstellung der Figur 5 dient lediglich der Veranschaulichung und spiegelt die Realität nicht vollständig wider, weil das Dielektrikum 2 mit den Schichten 3, 4 in aller Regel in einem einzigen Verfahrensschritt durch Umgießen der Elektrode 1 hergestellt werden wird, insbesondere, um innerhalb der Durchgangsöffnungen 11 der Elektrode 1 eine durchgehende und vollständige Isolierung durch das Dielektrikum 2 bis auf die kleinen Durchgangsöffnungen 12 zu gewährleisten. Grundsätzlich ist es allerdings auch denkbar, das Dielektrikum 2 aus zwei Schichten 3, 4 herzustellen, wenn beispielsweise auf Durchgangsöffnungen 11, 12 verzichtet werden soll, wenn eine Abführung von Wundsekret beispielsweise nicht erforderlich erscheint. Darüber hinaus ist es natürlich auch möglich, die beiden Schichten 3, 4 durch einen Spiegelschweißvorgang miteinander materialschlüssig zu verbinden, und zwar in dem rahmenförmigen Umfassungsbereich der Elektrode 2, aber auch durch die Durchgangsöffnungen 11 hindurch, unter Beibehaltung der Durchgangsöffnungen 12 des Dielektrikums 2.

Obwohl in den dargestellten Ausführungsbeispielen die Gitterstruktur 6 einstückig mit dem Dielektrikum 2 dargestellt ist, kann es vorteilhaft sein, die Gitterstruktur 6 separat herzustellen und an der unteren Schicht 3 des Dielektrikums 2 zu fixieren. Die Herstellung der Gitterstruktur kann durch Gießen mit Hilfe einer entsprechenden Form oder auch durch einen 3D-Druck erfolgen. Eine Fixierung der separat hergestellten Gitterstruktur 6 an der unteren Schicht 3 in einer leicht auswechselbaren Weise ermöglicht einen aus Sterilitätsgründen möglicherweise wünschenswerten Austausch der Gitterstruktur 6 unter Beibehaltung der Elektrodenanordnung im Übrigen.

Die Kontaktierung der Elektrodenanordnung zur Zuführung einer für die Plasmaerzeugung benötigten Hochspannung, die vorzugsweise als Wechselspannung verwendet wird, erfolgt mit einer maulartigen Anschlussklemme, die die in der Aussparung 5 freiliegende Fläche der Elektrode 1 sicher vollständig isoliert, sodass eine zufällige Berührung der mit Hochspannung versorgten Elektrode 1 ausgeschlossen ist. Eine geeignete Kontaktierungseinrichtung ist in WO 2012/175066 A1 beschrieben und zeichnerisch dargestellt, sodass hierauf Bezug genommen und auf eine erneute Beschreibung verzichtet werden kann.

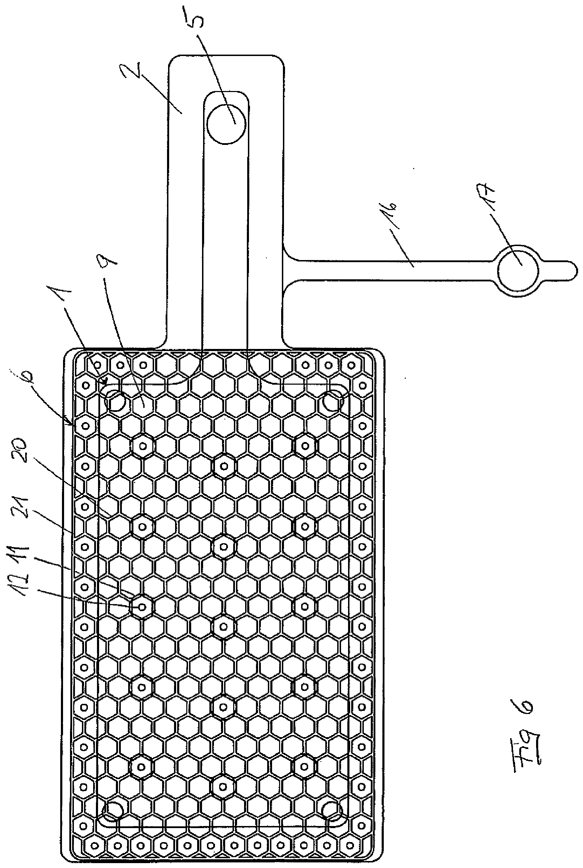

In den Figuren 6 bis 10 sind weitere Ausführungsformen einer erfindungsgemäßen Elektrodenanordnung dargestellt, die sich durch die Form der Struktur 6 unterscheiden.

Gemäß der in Figur 6 dargestellten dritten Ausführungsform sind die Kammern 9 der Struktur 6 wabenförmig, das heißt als gleichseitiges Sechseck, ausgebildet. Auf diese Weise schließen sich die Kammern 9 lückenlos aneinander an und sind durch Wände 20 einer konstanten Wandstärke voneinander getrennt. Die Struktur 6 ist durch eine umlaufende Abschlusswand 21 eingefasst. Auch in dieser Ausführungsform, die im Übrigen mit den ersten und zweiten Ausführungsformen übereinstimmt, können sich durch das Dielektrikum 2 Durchgangsöffnungen 12 erstrecken, die mit größeren Durchgangsöffnungen 11 in der flächigen Elektrode 1 fluchten.

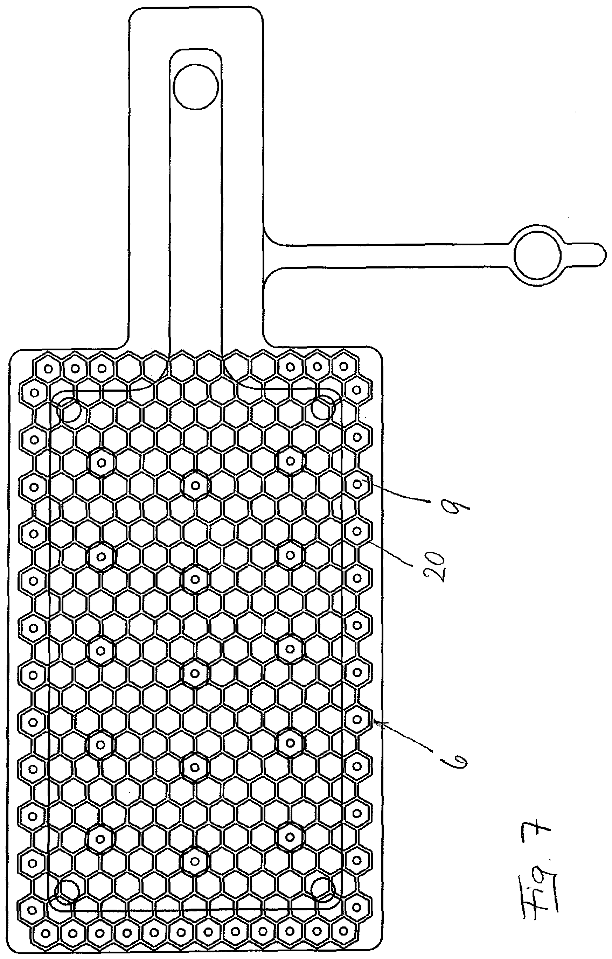

Die in Figur 7 dargestellte vierte Ausführungsform einer Elektrodenanordnung entspricht dem Aufbau der dritten Ausführungsform gemäß Figur 6, ist jedoch ohne eine umlaufende Abschlusswand 21 ausgebildet, sodass die Struktur 6 durch die mäanderförmigen Wände 20 der Kammern 9 am Rand der Struktur 6 begrenzt wird. Auch hier erstreckt sich die Struktur 6 in beiden flächigen Ausdehnungen über die jeweilige Erstreckung der Elektrode 1 hinaus, sodass die Struktur 6 die Elektrode 1 flächig allseitig überragt.

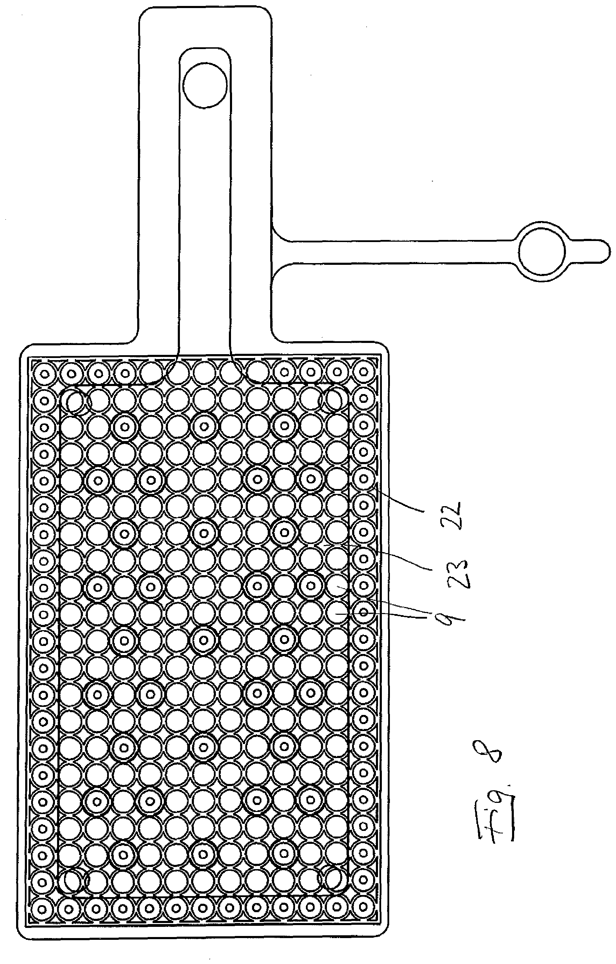

Bei der in Figur 8 dargestellten fünften Ausführungsform sind die Kammern 9 kreisrund ausgebildet und durch entsprechende hohlzylindrische Wände 22 begrenzt. Da die Wände 22 mit einer konstanten Wandstärke ausgebildet sind, entstehen zwischen den Kammern 9 Zwickelkammern 23, in denen sich ebenfalls ein Plasma ausbilden kann. Der übrige Aufbau der Elektrodenanordnung entspricht den vorhergehenden Ausführungsformen.

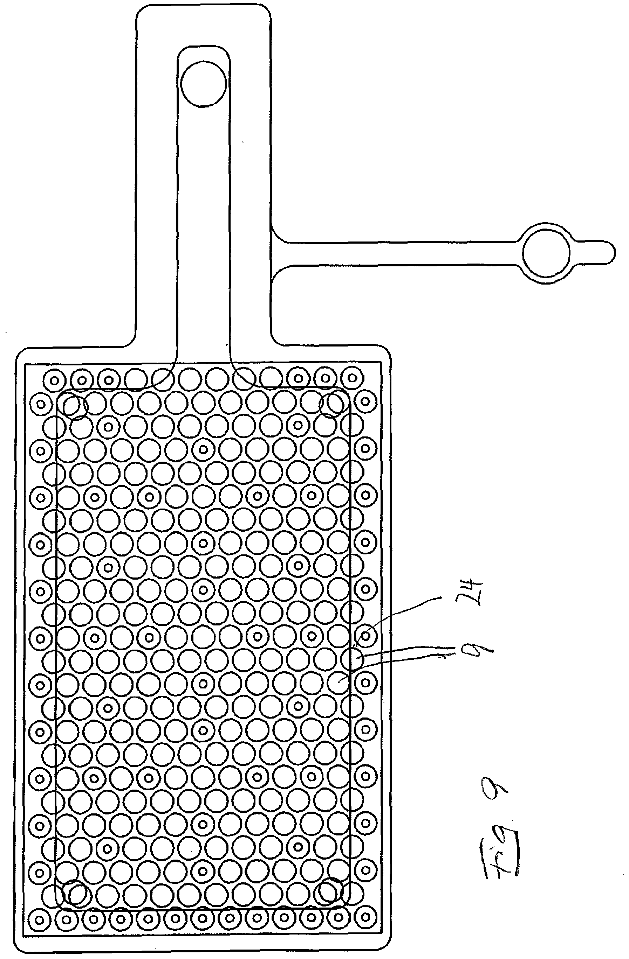

In der in Figur 9 dargestellten sechsten Ausführungsform sind die Kammern 9 ebenfalls kreisrund ausgebildet, jedoch von Wänden 24 begrenzt, die den Zwischenraum zwischen den Kammern 9 vollständig ausfüllen und somit entsprechend variierende Wandstärken aufweisen. In dieser Ausführungsform werden die Wände 24 durch mehr Material gebildet als in der Ausführungsform gemäß Figur 8. Der Mehrverbrauch an Material wird dadurch begrenzt, dass in der Ausführungsform gemäß Figur 9 die Kammern 9 einer Zeile bzw. Spalte zu den Kammern 9 der benachbarten Zeile oder Spalte um jeweils eine halbe Kammerbreite versetzt angeordnet sind, während in der Ausführungsform gemäß Figur 8 die Kammern waagerechte Zeilen bzw. vertikale Spalten bilden. Dem höheren Materialaufwand für die Ausführungsform gemäß Figur 9 steht eine vereinfachte Fertigung gegenüber.

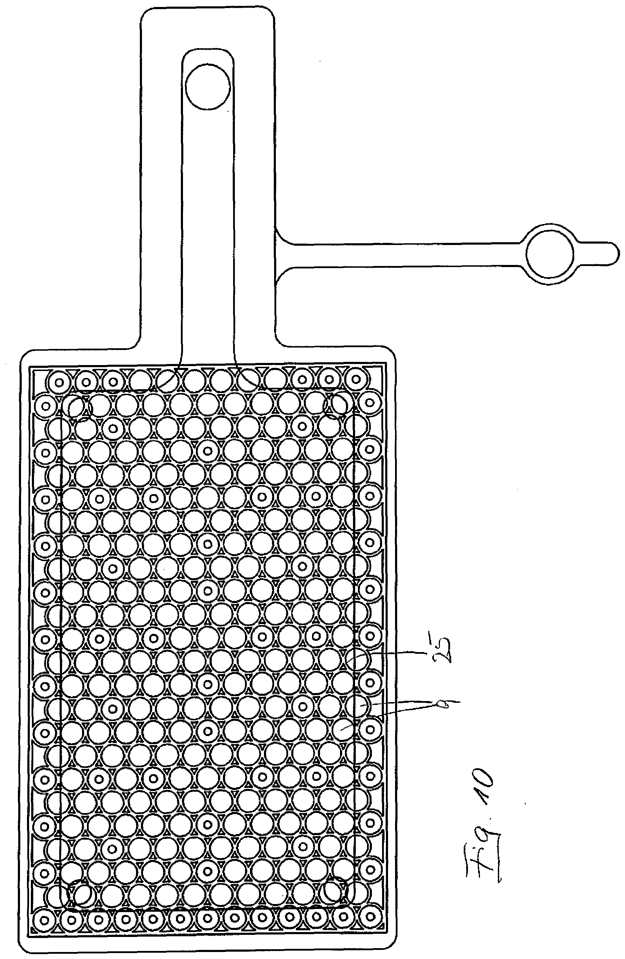

Bei der siebten Ausführungsform gemäß Figur 10 sind die kreisrunden Kammern 9, wie in der sechsten Ausführungsform gemäß Figur 9, benachbart versetzt angeordnet, wobei kleine Zwickelräume 25 entstehen, die durch ein massives dreieckförmiges Material oder einen entsprechenden dreieckförmigen Hohlzylinder ausgefüllt sein können. Der Aufbau der Elektrodenanordnung in dieser Ausführungsform entspricht im Übrigen dem der vorhergehenden Ausführungsformen.

Es ist ohne Weiteres ersichtlich, dass auch andere Strukturen 6 mit allseits geschlossenen Kammern 9 gebildet werden können, wobei die Kammern 9 auch in unterschiedlicher Größe ausgebildet sein können, wie dies gemäß Figur 8 bei den dort dargestellten Kammern 9 und 23 bereits der Fall ist.

## Patentansprüche

1. Elektrodenanordnung für eine dielektrisch behinderte Plasmabehandlung einer als Gegenelektrode verwendeten Oberfläche eines elektrisch leitenden Körpers, mit einer flexiblen flächigen Elektrode (1) und einem Dielektrikum (2) aus einem flächigen flexiblen Material, das mit einer einen direkten Stromfluss verhindernden Schicht (3) die Elektrode (1) von der zu behandelnden Oberfläche abschirmt, wobei das Dielektrikum (2) über eine Struktur mit Vorsprüngen, auf der zu behandelnden Oberfläche aufliegen kann und dabei zwischen den Vorsprüngen Lufträume für die Ausbildung des Plasmas ausgebildet sind, wobei die Struktur eine Gitterstruktur (6) ist, die Lufträume ausbildende Kammern (9) aufweist und die Kammern (9) einen bodenseitigen Abschluss durch die den direkten Stromfluss verhindernde Schicht (3) des Dielektrikums (2) und eine zur zu behandelnden Oberfläche offene Seite aufweisen, wobei die Anlagefläche an der zu behandelnden Oberfläche aus Endkanten (10) der Wände (7,8) der Gitterstruktur (6) besteht,
**dadurch gekennzeichnet, dass** die
Kammern (9) durch aneinander angrenzende Wände (7, 8) mit einer Materialstärke zwischen 0,1 und 1,0 mm und einer Höhe von 0,5 bis 3 mm über der den direkten Stromfluss verhindernden Schicht des Dielektrikums (2) begrenzt sind.

2. Elektrodenanordnung nach Anspruch 1, **dadurch gekennzeichnet, dass** die Gitterstruktur (6) aus winkelig zueinander stehenden Scharen von zahlreichen Wänden (7, 8) ist, von denen jeweils eine Kammer (9) durch jeweils zwei einander kreuzende Wandpaare (7, 8) begrenzt ist.

3. Elektrodenanordnung nach Anspruch 2, **dadurch gekennzeichnet, dass** die Wände (7, 8) in den Scharen zueinander parallel verlaufen.

4. Elektrodenanordnung nach Anspruch 3, **dadurch gekennzeichnet, dass** zwei Scharen von Wänden (7, 8) existieren, deren Wände (7, 8) senkrecht zueinander stehen.

5. Elektrodenanordnung nach Anspruch 4, **dadurch gekennzeichnet, dass** die Wände (7, 8) im Querschnitt quadratische Kammern (9) begrenzen.

6. Elektrodenanordnung nach Anspruch 1, **dadurch gekennzeichnet, dass** Kammern (9) mit einem runden, ovalen oder vieleckigen, insbesondere wabenförmigen, Querschnitt gebildet sind.

7. Elektrodenanordnung nach einem der Ansprüche 1 bis 6, **dadurch gekennzeichnet, dass** die Materialstärke der Wände (7, 8) weniger als 20 % der größten Breite einer Kammer (9) ausmacht.

8. Elektrodenanordnung nach Anspruch 7, **dadurch gekennzeichnet, dass** die Materialstärke der Wände (7, 8) weniger als 10 % der größten Breite einer Kammer (9) ausmacht.

9. Elektrodenanordnung nach einem der Ansprüche 1 bis 8, **dadurch gekennzeichnet, dass** die Gitterstruktur (6) einstückig mit der den direkten Stromfluss verhindernden Schicht (3) des Dielektrikums (2) ausgebildet ist.

10. Elektrodenanordnung nach einem der Ansprüche 1 bis 9, **dadurch gekennzeichnet, dass** die Gitterstruktur (6) als separates Teil gefertigt und an die den direkten Stromfluss verhindernde Schicht (3) des Dielektrikums (2) angesetzt ist.

11. Elektrodenanordnung nach einem der Ansprüche 1 bis 10, **dadurch gekennzeichnet, dass** die flächige Elektrode (1) über ihre Fläche verteilt Durchgangsöffnungen (11) aufweist und dass sich das die Elektrode (1) abschirmende Dielektrikum (2) auf beiden Seiten der flächigen Elektrode (1) erstreckt und mit zur Ableitung von Fluid von der zu behandelnden Oberfläche ausgebildeten Durchgangsöffnungen (12) versehen ist, die mit den Durchgangsöffnungen (11) der Elektrode (1) fluchten und kleinere Abmessungen als die Durchgangsöffnungen (11) der Elektrode (1) aufweisen, sodass das Dielektrikum (2) auch im Bereich der Durchgangsöffnungen (11) die Elektrode (1) vollständig abdeckt.

12. Elektrodenanordnung nach Anspruch 11, **dadurch gekennzeichnet, dass** die Durchgangsöffnungen (12) der den direkten Stromfluss verhindernden Schicht (3) mit Kammern (9) der Gitterstruktur (6) fluchten.

13. Elektrodenanordnung nach einem der Ansprüche 1 bis 12, **dadurch gekennzeichnet, dass** die Struktur (6) aus einem dielektrischen Material besteht.

## Claims

1. An electrode arrangement for a dielectric barrier discharge plasma treatment of a surface of an electrically conductive body used as a counterelectrode, with a flexible planar electrode (1) and with a dielectric (2) which is composed of a planar flexible material and which, with a layer (3) preventing a direct current flow, shields the electrode (1) from the surface to be treated, wherein the dielectric (2), by way of a structure with projections, can rest on the surface to be treated, and wherein air spaces for the formation of the plasma are formed between the projections, the structure being a lattice structure (6) having chambers (9) forming air spaces, the chambers (9) being closed off at the bottom by the layer (3) of the dielectric preventing the direct current flow, and having a side which is open to the surface to be treated, wherein the contact face for bearing on the surface to be treated consists of end edges (10) of the walls (7, 8) of the lattice structure (6), **characterized in that** the chambers are delimited by mutually adjoining walls (7, 8) having a material thickness between 0.1 and 1.0 mm and a height of 0.5 to 3.0 mm above the layer (3) of the dielectric (2) preventing the direct current flow.

2. The electrode arrangement as claimed in claim 1, **characterized in that** the lattice structure (6) is composed of sets of numerous walls (7, 8) which are at an angle to one another, of which one chamber (9) is in each case delimited by two intersecting wall pairs (7, 8).

3. The electrode arrangement as claimed in claim 2, **characterized in that** the walls (7, 8) in the sets extend parallel to one another.

4. The electrode arrangement as claimed in claim 3, **characterized in that** two sets of walls (7, 8) exist whose walls (7, 8) are perpendicular to one another.

5. The electrode arrangement as claimed in claim 4, **characterized in that** the walls (7, 8) in cross section delimit square chambers (9).

6. The electrode arrangement as claimed in claim 1, **characterized in that** chambers (9) are formed with a round, oval or polygonal cross section, in particular a honeycombed cross section.

7. The electrode arrangement as claimed in one of claims 1 through 6, **characterized in that** the material thickness of the walls (7, 8) makes up less than 20% of the greatest width of a chamber (9) .

8. The electrode arrangement as claimed in claim 7, **characterized in that** the material thickness of the walls (7, 8) makes up less than 10% of the greatest width of a chamber (9).

9. The electrode arrangement as claimed in one of claims 1 through 8, **characterized in that** the lattice structure (6) is formed in one piece with the layer (3) of the dielectric (2) preventing the direct current flow.

10. The electrode arrangement as claimed in one of claims 1 through 9, **characterized in that** the lattice structure (6) is produced as a separate part and is joined to the layer (3) of the dielectric (2) preventing the direct current flow.

11. The electrode arrangement as claimed in one of claims 1 through 10, **characterized in that** the planar electrode (1) has through-openings (11) distributed across its surface, and **in that** the dielectric (2) shielding the electrode (1) extends on both sides of the planar electrode (1) and is provided with through-openings (12) designed for draining fluid from the surface to be treated, said through-openings (12) being in alignment with the through-openings (11) of the electrode (1) and having smaller dimensions than the through-openings (11) of the electrode (1), such that the dielectric (2) also completely covers the electrode (1) in the region of the through-openings (11).

12. The electrode arrangement as claimed in claim 11, **characterized in that** the through-openings (12) of the layer (3) preventing the direct current flow are in alignment with chambers (9) of the lattice structure (6).

13. The electrode arrangement as claimed in one of claims 1 through 12, **characterized in that** the structure (6) is composed of a dielectric material.

## Revendications

1. Ensemble d'électrode pour un traitement d'une surface d'un corps électriquement conducteur avec un plasma empêché par voie diélectrique, ladite surface faisant office de contre-électrode, comportant une électrode (1) surfacique flexible et un diélectrique (2) d'un matériau surfacique flexible qui protège l'électrode (1) vis-à-vis de la surface à traiter par une couche (3) empêchant une circulation de courant directe, le diélectrique (2) pouvant reposer sur la surface à traiter par une structure présentant des protubérances, des espaces d'air pour réaliser le plasma étant ménagés entre les protubérances, la structure étant une structure en grille (6) qui présente des chambres (9) formant des espaces d'air, et les chambres (9) présentant une terminaison côté fond par la couche (3) du diélectrique (2) empêchant la circulation de courant directe, et présentant un côté ouvert vers la surface à traiter, la surface d'appui contre la surface à traiter étant constituée par des arêtes d'extrémité (10) des parois (7, 8) de la structure en grille (6),
**caractérisé en ce que**
les chambres (9) sont délimitées par des parois adjacentes (7, 8) d'une épaisseur comprise entre 0,1 et 1,0 mm et d'une hauteur de 0,5 à 3 mm au-dessus de la couche du diélectrique (2) empêchant la circulation de courant directe.

2. Ensemble d'électrode selon la revendication 1,
**caractérisé en ce que**
la structure en grille (6) est constituée par des groupes de nombreuses parois (7, 8) disposées en angle les unes par rapport aux autres, dont une chambre respective (9) est délimitée par deux paires de parois (7, 8) qui se croisent.

3. Ensemble d'électrode selon la revendication 2,
**caractérisé en ce que**
les parois (7, 8) dans les groupes s'étendent parallèlement les unes aux autres.

4. Ensemble d'électrode selon la revendication 3,
**caractérisé en ce que**
il existe deux groupes de parois (7, 8), dont les parois (7, 8) sont perpendiculaires les unes aux autres.

5. Ensemble d'électrode selon la revendication 4,
**caractérisé en ce que**
les parois (7, 8) délimitent des chambres (9) de section transversale carrée.

6. Ensemble d'électrode selon la revendication 1,
**caractérisé en ce que**
les chambres (9) sont formées avec une section transversale ronde, ovale ou polygonale, en particulier en forme de nids d'abeilles.

7. Ensemble d'électrode selon l'une des revendications 1 à 6,
**caractérisé en ce que**
l'épaisseur des parois (7, 8) fait moins de 20 % de la plus grande largeur d'une chambre (9).

8. Ensemble d'électrode selon la revendication 7,
**caractérisé en ce que**
l'épaisseur des parois (7, 8) fait moins de 10 % de la plus grande largeur d'une chambre (9).

9. Ensemble d'électrode selon l'une des revendications 1 à 8,
**caractérisé en ce que**
la structure en grille (6) est réalisée d'un seul tenant avec la couche (3) du diélectrique (2) empêchant la circulation de courant directe.

10. Ensemble d'électrode selon l'une des revendications 1 à 9,
**caractérisé en ce que**
la structure en grille (6) est fabriquée comme une pièce séparée et est rapportée à la couche (3) du diélectrique (2) empêchant la circulation de courant directe.

11. Ensemble d'électrode selon l'une des revendications 1 à 10,
**caractérisé en ce que**
l'électrode surfacique (1) présente des ouvertures de passage (11) réparties sur sa surface, et **en ce que**
le diélectrique (2) protégeant l'électrode (1) s'étend de part et d'autre de l'électrode surfacique (1) et est pourvu d'ouvertures de passage (12) ménagées pour évacuer du fluide depuis la surface à traiter, ouvertures qui sont en alignement avec les ouvertures de passage (11) de l'électrode (1) et qui présentent des dimensions inférieures à celles des ouvertures de passage (11) de l'électrode (1), de sorte que le diélectrique (2) recouvre complètement l'électrode (1) également au niveau des ouvertures de passage (11).

12. Ensemble d'électrode selon la revendication 11,
**caractérisé en ce que**
les ouvertures de passage (12) de la couche (3) empêchant la circulation de courant directe sont en alignement avec des chambres (9) de la structure en grille (6).

13. Ensemble d'électrode selon l'une des revendications 1 à 12,
**caractérisé en ce que**
la structure (6) est constituée d'un matériau diélectrique.
